# EUROPEAN PATENT SPECIFICATION

(11) **EP 2 707 498 B1**
(45) Date of publication and mention of the grant of the patent: **17.01.2018**
(21) Application number: 12702740.7
(22) Date of filing: 30.01.2012
(51) Int. Cl.: C12Q 1/68, G06F 19/18

(54) **METHOD FOR DISCOVERING PHARMACOGENOMIC BIOMARKERS**
VERFAHREN ZUR ENTDECKUNG VON PHARMAKOGENOMISCHEN BIOMARKERN
PROCÉDÉ DE DÉCOUVERTE DE BIOMARQUEURS PHARMACOGÉNOMIQUES

(30) Priority: 31.01.2011 US 201161437788 P
(43) Date of publication of application: 19.03.2014
(73) Proprietor: Denovo Biomarkers Inc., San Diego, CA 92121 (US)
(72) Inventor: LUO, Wen, San Diego, CA 92130 (US)
(74) Representative: Witte, Weller & Partner Patentanwälte mbB
(86) International application number: PCT/US2012/023195
(87) International publication number: WO 2012/106267

(56) References cited:
- US-B1- 7 790 396
- JONATHAN B SINGER ET AL: "A genome-wide study identifies HLA alleles associated with lumiracoxib-related liver injury", NATURE GENETICS, vol. 42, no. 8, 1 August 2010 (2010-08-01) , pages 711-714, XP55026364, ISSN: 1061-4036, DOI: 10.1038/ng.632 cited in the application
- MEAD S ET AL: "Genetic risk factors for variant Creutzfeldt-Jakob disease: a genome-wide association study", LANCET NEUROLOGY, LANCET PUBLISHING GROUP, LONDON, GB, vol. 8, no. 1, 1 January 2009 (2009-01-01) , pages 57-66, XP025838467, ISSN: 1474-4422, DOI: 10.1016/S1474-4422(08)70265-5 [retrieved on 2008-12-24]
- ANDREW D SKOL ET AL: "Joint analysis is more efficient than replication-based analysis for two-stage genome-wide association studies", NATURE GENETICS, vol. 38, no. 2, 1 February 2006 (2006-02-01), pages 209-213, XP55026798, ISSN: 1061-4036, DOI: 10.1038/ng1706
- HOLLEGAARD MADS V ET AL: "Genome-wide scans using archived neonatal dried blood spot samples", BMC GENOMICS, BIOMED CENTRAL, LONDON, GB, vol. 10, no. 1, 4 July 2009 (2009-07-04), page 297, XP021056107, ISSN: 1471-2164, DOI: 10.1186/1471-2164-10-297 cited in the application
- HOLLEGAARD MADS V ET AL: "High-Throughput Genotyping on Archived Dried Blood Spot Samples", GENETIC TESTING AND MOLECULAR BIOMARKERS, vol. 13, no. 2, April 2009 (2009-04), pages 173-179, XP55026723, ISSN: 1945-0257, DOI: 10.1089=gtmb.2008.0073
- HABIBA S AL SAFAR ET AL: "Evaluation of different sources of DNA for use in genome wide studies and forensic application", APPLIED MICROBIOLOGY AND BIOTECHNOLOGY, SPRINGER, BERLIN, DE, vol. 89, no. 3, 27 October 2010 (2010-10-27), pages 807-815, XP019874913, ISSN: 1432-0614, DOI: 10.1007/S00253-010-2926-3
- BUCASAS KRISTINE L ET AL: "Assessing the utility of whole-genome amplified serum DNA for array-based high throughput genotyping", BMC GENETICS, BIOMED CENTRAL, GB, vol. 10, no. 1, 18 December 2009 (2009-12-18), page 85, XP021065951, ISSN: 1471-2156
- R. M. SIMON ET AL: "Use of Archived Specimens in Evaluation of Prognostic and Predictive Biomarkers", JNCI JOURNAL OF THE NATIONAL CANCER INSTITUTE, vol. 101, no. 21, 4 November 2009 (2009-11-04), pages 1446-1452, XP55026477, ISSN: 0027-8874, DOI: 10.1093/jnci/djp335
- LASKEN R S ET AL: "Whole genome amplification: abundant supplies of DNA from precious samples or clinical specimens", TRENDS IN BIOTECHNOLOGY, ELSEVIER PUBLICATIONS, CAMBRIDGE, GB, vol. 21, no. 12, 1 December 2003 (2003-12-01), pages 531-535, XP004473517, ISSN: 0167-7799, DOI: 10.1016/J.TIBTECH.2003.09.010
- LUO WEN ET AL: "Identification of polymorphisms associated with hypertriglyceridemia and prolonged survival induced by bexarotene in treating non-small cell lung cancer.", ANTICANCER RESEARCH JUN 2011 LNKD- PUBMED:21737656, vol. 31, no. 6, June 2011 (2011-06), pages 2303-2311, XP009159180, ISSN: 1791-7530
- LU YANHUI ET AL: "Use of whole genome amplification to rescue DNA from plasma samples", BIOTECHNIQUES, INFORMA HEALTHCARE, US, vol. 39, no. 4, 1 October 2005 (2005-10-01), pages 511-515, XP001538765, ISSN: 0736-6205, DOI: 10.2144/000112005
- Karina Meden Sørensen ET AL: "Whole Genome Amplification on DNA from Filter Paper Blood Spot Samples: An Evaluation of Selected Systems", Genetic Testing, vol. 11, no. 1, 1 April 2007 (2007-04-01), pages 65-71, XP055166152, ISSN: 1090-6576, DOI: 10.1089/gte.2006.0503
- DANIEL T. CROFT ET AL: "Performance of Whole-Genome Amplified DNA Isolated from Serum and Plasma on High-Density Single Nucleotide Polymorphism Arrays", JOURNAL OF MOLECULAR DIAGNOSTICS,THE, vol. 10, no. 3, 1 May 2008 (2008-05-01), pages 249-257, XP055242010, US ISSN: 1525-1578, DOI: 10.2353/jmoldx.2008.070155
- Stephen Turner ET AL: "Quality Control Procedures for Genome-Wide Association Studies" In: "Current Protocols in Human Genetics", 1 January 2011 (2011-01-01), John Wiley & Sons, Inc., Hoboken, NJ, USA, XP055241966, ISSN: 1934-8266 ISBN: 978-0-471-14290-4 pages 1.19.1-1.19.18, DOI: 10.1002/0471142905.hg0119s68,

## Description

### Technical Field

The present invention relates to a method to identify and verify one or more pharmacogenomic biomarkers which can be developed into companion diagnostic tests to predict varied individual responses (efficacy, adverse effect, or other) to therapeutic agents.

### Background Art

Pharmaceutical industry has been operating under the paradigm of "one drug fits all" for many decades. However, only a few drugs offer universal efficacy in all patient populations, and some even cause serious adverse effects in certain patient groups. These obstacles, in addition to sky rocketing R&D expenses and the tougher FDA review standards, have resulted in many of newly developed drugs failing to reach the market. Therefore, identifying pharmacogenomic biomarkers, which can predict potential responders for a drug, would be the ideal solution to unlock the hidden value for many of these otherwise failed drugs.

In the meantime, with the completion of HapMap project and rapid advances in microarray technology, genome-wide scan of genetic polymorphisms has become routine tasks, and hundreds of genome wide association studies (GWAS) have been successfully conducted and led to the discovery of a large number of known and novel genetic variants associated with common diseases such as cardiovascular disease, diabetes, etc. This technology is also offering powerful tools for identifying genetic polymorphisms associated with drag responses. However, Guessous et al. reviewed about three hundred GWAS studies, among which only 12 are pharmacogenomic studies (Genome Med (2009) 1:46). Of those 12 studies, only two of them are GWAS used in clinical trials. Obviously, the pharmaceutical companies embraced this new technology at a very slow pace comparing to academic
researchers. One of the most common causes is that pharmaceutical companies only realize the need to perform such study after the clinical trials are over, and it is often too late at this point since the appropriate samples have not been collected during the trial. This scenario will likely remain unchanged for the foreseeable future until the industry widely adopts the concept of pharmacogenomics and begins to incorporate biomarker design prior to starting clinical trials.

Despite increased spending and advances in technology, a large number of newly developed drugs would fail in Phase III clinical trials, mostly due to lack of significant efficacy in overall patient population or unsatisfied safety profile. However, many of the failed drugs could still benefit a subset of patient population or only cause adverse effects in small number of patients. Comparing to the "old" candidate gene approach, GWAS is hypothesis free and doesn't require prior knowledge of the mechanisms involved in the studied clinical end points. The advances in array technology have also made it possible to genotype 1 million or more SNPs, which could cover the entire human genome, on a single array. Therefore, GWAS is an ideal option to search drug specific pharmacogenomic biomarkers.

The commonly used sources of genomic DNA in GWAS are DNA rich tissues/cells such as whole blood, which can yield adequate amount of high quality genomic DNA. However, most of the pharmaceutical companies have not incorporated pharmacogenomic study into their clinical trials. Thus there are usually no dedicated samples collected for GWAS study. In many cases, there could be human specimen collected for other purposes, such as biopsies (for pathology) and plasma samples (for pharmacokinetic study). Those leftover clinical samples could be potentially used to obtain genomic DNA. A few reports have shown that genomic DNA could be extracted from plasma samples and lead to successful genotyping on a small number of SNPs (Sjoholm et al., Cancer Epidemiol Biomarkers Prev (2005) 14:251; Lu et al., Biotechniques (2005) 39:511; Park et al., Clin Chem (2005) 51:1520; Bergen et al., Hum Mutat (2005) 26:262). However, the quantity and quality of the genomic DNA from archived clinical samples, which are often being processed and stored inappropriately, are far from optimal, especially for usage in GWAS. In fact, among the few who have published reports on using genomic DNA extracted from plasma samples on high density SNP arrays, the authors suggested that the DNA quality was so "poor" from these samples and they might not lead to a successfully GWAS (Croft et al., J Mol Diagn (2008) 10:249; Bucasas et al., BMC Genet (2009) 10:85). The only GWAS successfully conducted used dried blood spot samples (Hollegaard et al, BMC Genomics (2009) 10:297-302) or patient blood (Singer et al., Nat Genet (2010) 42:711-714). Thus, to our knowledge, no one has ever attempted to perform a successful GWAS using genomic DNA from archived clinical samples.
Singer et al., Nat Genet (2010) 42:711, disclose the results of a genome-wide study to identify HLA alleles associated with lumiracoxib-related liver injury. Mead et al., Lancet Neurol (2009) 8:57, disclose the results of a genome-wide association study on genetic risk factors for variant Creutzfeld-Jakob disease. US 7,790,396 discloses methods and compositions for the treatment of psychotic disorders through the identification of the SULT4A1-1 haplotype. Luo et al., Anticancer Res (2011) 31:2303, relates to the identification of polymorphisms associated with hypertriglyceridemia and prolonged survival induced by bexarotene in treating non-small cell lung cancer. Bucasas et al., BMC Genet (2009) 10:85 were assessing the utility of whole-genome amplified serum DNA for array-based high throughput genotyping. Croft et al., J Mol Diagn (2008) 10:249, analyzed the performance of whole-genome amplified DNA isolated from serum and plasma on high-density single nucleotide polymorphism arrays.

### Summary of the Invention

While most of the published GWAS have focused on discovering the causative genetic variants of common diseases using high quality DNA from dedicated samples such as whole blood in well-designed studies, the application of this powerful technology has seldom been incorporated into clinical trials for new drugs and therefore has very limited successes in pharmacogenomic study. The current consensus is that a successful GWAS can only be performed using abundant high quality genomic DNA, and suboptimal genomic DNA and/or whole genome amplified DNA are strongly discouraged or abandoned in GWAS. The present invention describes a method of discovery of pharmacogenomic biomarkers by GWAS to predict drug response utilizing suboptimal genomic DNA, e.g., genomic DNA extracted from archived clinical samples.

In one aspect, the present invention provides a method to identify one or more pharmacogenomic biomarkers, which method comprises steps (a)-(g) as recited in claim 1.

The archived clinical samples are plasma samples.

The isolated DNA is suboptimal genomic DNA. The amplification is whole-genome amplification (WGA), and the resulting DNA is whole-genome amplified DNA (wgaDNA). The high-density genotyping may be whole-genome genotyping. The high-density genotyping may be conducted by using single nucleotide polymorphisms (SNPs). About 1,000-5,000,000 or more, preferably about 1,000,000, SNPs may be used for the high-density genotyping. The high-density genotyping may be array-based.

The genotyping data is obtained by using a genome- wide genotype calling algorithm. An optimal inclusion criterion for the genome-wide genotype calling algorithm may be identified. The genotype calls may be made by using a call rate cut-off value that is lower than a typical call rate cut-off used for whole-genome genotyping of high quality genomic DNA, wherein the call rate cut-off value used may be about 50%, 60%, 70%, 80%, or 90%. The genotype calls may be generated with the Affymetrix Genotyping Console™ software. The genotype calls may be generated using the BRLMM algorithm. In some embodiments, the genotype calls may be made using an imputation algorithm, wherein the HapMap may be used for the imputation algorithm.

The association analysis may be a GWAS. The association analysis may be performed by calculating an associated p-value of each SNP with the relevant phenotype. The calculation may be based on an allele frequency and/or genotype-based test. The relevant phenotype may be a categorical trait, a quantitative trait or another relevant phenotype.

The method further comprises performing association analysis based on additional genotyping data using the identified pharmacogenomic biomarkers. About 1-500 or more of the identified pharmacogenomic biomarkers may be used for the additional genotyping. Some or all of the archived clinical samples from step a) and/or additional clinical samples may be used for the additional genotyping. Additional genotyping data are obtained by using a verification genotype calling algorithm. The method further comprises adjusting the call rate cut-off value of the genotype calling algorithm. Genotyping and adjusting the call rate cut-off is repeated multiple times to include and/or exclude samples. The method further comprises comparing the additional genotyping data obtained by using the verification genotype calling algorithm to the genotyping data obtained by using the genome-wide genotype calling algorithm. A subset of the pharmacogenomic biomarkers from step d) is identified.

In some embodiments, the method may be used for retrospective study of archived clinical samples from a previously conducted clinical trial. The method may be used for *de novo* identification of a pharmacogenomic biomarker.

Provided herein is a pharmacogenomic biomarker, or a group of pharmacogenomic biomarkers, identified by the method disclosed herein, wherein the biomarker may be one or more SNPs. The pharmacogenomic biomarker may be used to identify one or more additional pharmacogenomic biomarkers. The pharmacogenomic biomarker may be used to develop a companion diagnostic test.

Provided herein is a companion diagnostic test using the pharmacogenomic biomarkers identified by the method disclosed herein. Also provided herein is a method of prognosticating responsiveness of a subject to a treatment using the companion diagnostic test disclosed herein. Further provided herein is a method of identifying a novel drug target using the pharmacogenomic biomarkers identified by the method disclosed herein. The method of the present invention is useful for clinicians to identify patients for treatment, aid in patient selection during the course of development of therapy, predict likelihood of success when treating an individual patient with a particular treatment regimen, assess and monitor disease progression, monitor treatment efficacy, and determine prognosis for individual patients.

Provided herein is a kit comprising a reagent for assessing the pharmacogenomic biomarker, or the group of pharmacogenomic biomarkers, identified by the method disclosed herein. The kit may further comprise instructions for using the pharmacogenomic biomarker to conduct a companion diagnostic test.

Provided herein is a genotyping method using suboptimal genomic DNA samples, which comprises the steps of: a) receiving sequence information of said suboptimal genomic DNA samples; b) optimizing an inclusion criterion based on said sequence information; and c) calculating genotypes based on said sequence information and said optimized inclusion criterion. The optimization may be repeated multiple times to include and/or exclude samples. An optimal inclusion criterion may be identified. The genotyping data may be obtained by using a genome-wide genotype calling algorithm and/or a verification genotype calling algorithm. The inclusion criterion may be the call rate cut-off value of the genotype calling algorithm. The genotype calls may be made by using a call rate cut-off that is lower than a typical call rate cut-off used for whole- genome genotyping of high quality or optimal genomic DNA, wherein the call rate cut-off value used may be about 50%, 60%, 70%, 80%, 90% or 95%. The genotyping data may be obtained by using multiple genotyping platforms. The genotyping data from multiple genotyping platforms may be compared for the optimization.

Further provided is a method to perform association analysis using the genotyping method using suboptimal genomic DNA samples, which method comprises optimizing an inclusion criterion. The association analysis may be repeated multiple times for the optimization. Also provided herein is a computer readable medium comprising a plurality of instructions for a genotyping method using suboptimal genomic DNA samples, which comprises the steps of: a) receiving sequence information of said suboptimal genomic DNA samples; b) optimizing an inclusion criterion based on said sequence information; and c) calculating genotypes based on said sequence information and said optimized inclusion criterion.

Provided herein is a method to conduct GWAS using suboptimal genomic DNA. The suboptimal genomic DNA may be from archived samples, The suboptimal genomic DNA may be from plasma samples. The suboptimal genomic DNA may be amplified. Multiple genotyping platforms may be used. The same or different samples may be used for the multiple genotyping platforms. The method may further use a sample providing high-quality genomic DNA.

### Brief Description of the Drawings

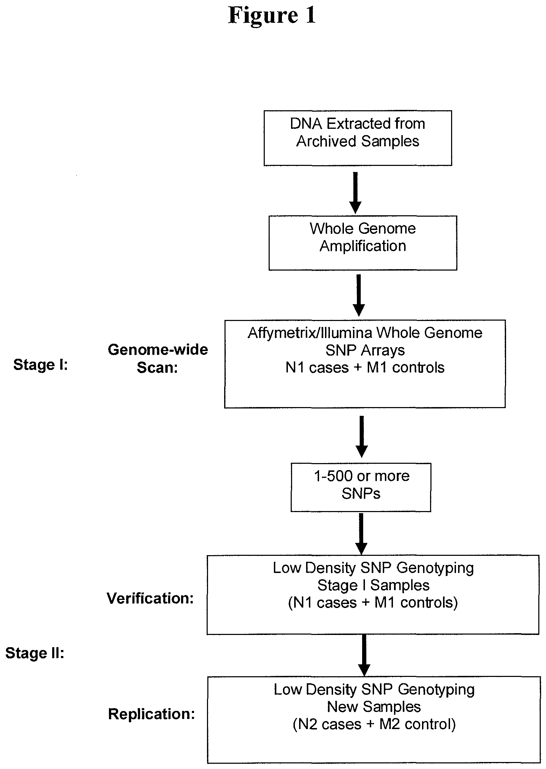

FIG. 1 shows the flow chart of an exemplary pharmacogenomic biomarker discovery method using GWAS. Genomic DNA is extracted from archived clinical samples, and amplified using WGA. In Stage I, the discovery phase, N1 (a number from 1 to 1,000 or more) samples from the case group and M1 (a number from 1 to 1,000 or more) samples from the control group are genotyped using the Affymetrix and/or Illumina whole genome SNP arrays. The association of each SNP to case-control status (i.e., responders vs. non-responders) is calculated based on an allele frequency and/or genotype-based test. Subsequently, 1 to 500 or more most significantly associated SNPs are selected for the Stage II study. In Stage II, the same samples used in Stage I are genotyped using a low density SNP genotyping platform (a distinctive genotyping technology from the ones used in Stage I) to verify the results obtained in Stage I. Additional new samples, N2 (a number from 1 to 1,000 or more) samples from the case group and M2 (a number from 1 to 1,000 or more) samples from the control group are genotyped to replicate the finding.
FIG. 2 shows the flow chart of an exemplary genome-wide discovery stage data analysis.
FIG. 3 shows the flow chart of an exemplary verification stage data analysis.

### Detailed Description of the Invention

The present invention provides novel approaches to overcome the potentially low quantity of genotyping results using genomic DNA isolated from archived clinical samples by applying more than one genotyping technology or platform.

### A. General Techniques

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al, eds., 1987, and periodic updates); "PCR: The Polymerase Chain Reaction", (Mullis et al, eds., 1994).

### B. Definitions

Unless defined otherwise, all technical and scientific terms used herein have the same meaning as is commonly understood by one of ordinary skill in the art to which this invention belongs. If a definition set forth in this section is contrary to or otherwise inconsistent with a definition set forth in the patents, applications, published applications and other publications that are herein quoted, the definition set forth in this section prevails over the definition that is quoted.

As used herein, the singular forms "a", "an", and "the" include plural references unless indicated otherwise. For example, "a" dimer includes one or more dimers.

The term "biomarker" or "marker" as used herein refers generally to a molecule, including a gene, protein, carbohydrate structure, or glycolipid, the expression of which in or on a mammalian tissue or cell or secreted can be detected by known methods (or methods disclosed herein) and is predictive or can be used to predict (or aid prediction) for a mammalian cell's or tissue's sensitivity to, and in some embodiments, to predict (or aid prediction) an individual's responsiveness to treatment regimens.

As used herein, a "pharmacogenomic biomarker" is an objective biomarker which correlates with a specific clinical drug response or susceptibility in a subject (*see, e.g.,* McLeod et al, Eur. J. Cancer (1999) 35: 1650-1652). It may be a biochemical biomarker, a clinical sign or symptom, etc. The presence or quantity of the pharmacogenomic marker is related to the predicted response of the subject to a specific drag or class of drags prior to administration of the drag. By assessing the presence or quantity of one or more pharmacogenomic markers in a subject, a drug therapy which is most appropriate for the subject, or which is predicted to have a greater degree of success, may be selected. For example, based on the presence or quantity of DNA, RNA, or protein for specific tumor markers in a subject, a drag or course of treatment may be selected that is optimized for the treatment of the specific tumor likely to be present in the subject. Similarly, the presence or absence of a specific sequence mutation or polymorphism may correlate with drag response.
The use of pharmacogenomic biomarkers therefore permits the application of the most appropriate treatment for each subject without having to administer the therapy.

The term "sample", as used herein, refers to a composition that is obtained or derived from a subject of interest that contains a cellular and/or other molecular entity that is to be characterized and/or identified, for example based on physical, biochemical, chemical and/or physiological characteristics. For example, the phrase "clinical sample" or "disease sample" and variations thereof refer to any sample obtained from a subject of interest that would be expected or is known to contain the cellular and/or molecular entity, such as a biomarker, that is to be characterized.

The term "tissue or cell sample" refers to a collection of similar cells obtained from a tissue of a subject or patient. The source of the tissue or cell sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. The tissue sample may contain compounds which are not naturally intermixed with the tissue in nature such as preservatives, anticoagulants, buffers, fixatives, nutrients, antibiotics, or the like.

"Plasma," or "blood plasma," as used herein, refers to the intravascular fluid part of extracellular fluid (all body fluid outside of cells). It is mostly water and contains dissolved proteins, glucose, clotting factors, mineral ions, hormones and carbon dioxide (plasma being the main medium for excretory product transportation). Blood plasma is prepared by spinning a tube of fresh blood containing an anti-coagulant in a centrifuge until the blood cells fall to the bottom of the tube. The blood plasma is then poured or drawn off. "Blood serum" is blood plasma without fibrinogen or the other clotting factors (i.e., whole blood minus both the cells and the clotting factors).

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as urmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-2'-O- allyl, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR 2 ("amidate"), P(O)R, P(O)OR', CO or CH 2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The term "suboptimal genomic DNA" as used herein refers to genomic DNA that is inferior in quality and/or in quantity to genomic DNA isolated from DNA rich tissues/cells such as whole blood. Suboptimal genomic DNA may be isolated from archived clinical samples such as biopsies or plasmas, which are often being processed and stored inappropriately, and are far from optimal, in quality and/or in quantity, especially for usage in GWAS. For example, a sample of suboptimal genomic DNA may not provide full coverage of the whole genome, or may contain short fragments of genomic DNA. A sample of suboptimal genomic DNA subjected to high-density genotyping may have a call rate of less than about 99%, 95%, 90%, 80%, 70%, 60%, 50% or lower. Typically, for the suboptimal genomic DNA to be useful for GWAS, an amplification step is needed.

"Amplification," as used herein, generally refers to the process of producing multiple copies of a desired sequence. "Multiple copies" means at least 2 copies. A "copy" does not necessarily mean perfect sequence complementarity or identity to the template sequence. For example, copies can include nucleotide analogs such as deoxyino-sine, intentional sequence alterations (such as sequence alterations introduced through a primer comprising a sequence that is hybridizable, but not complementary, to the template), and/or sequence errors that occur during amplification.

The term "array" or "microarray", as used herein refers to an ordered arrangement of hybridizable array elements, such as polynucleotide probes (e.g., oligonucleotides), or binding reagents (e.g., antibodies), on a substrate. The substrate can be a solid substrate, such as a glass or silica slide, a bead, a fiber optic binder, or a semi-solid substrate, such as a nitrocellulose membrane. The nucleotide sequences can be DNA, RNA, or any permutations thereof.

As used herein, the term "phenotype" refers to a trait which can be compared between individuals, such as presence or absence of a condition, a visually observable difference in appearance between individuals, metabolic variations, physiological variations, variations in the function of biological molecules, and the like. A phenotype can be qualitative or quantitative. An example of a phenotype is responsiveness to a treatment, such as a drug.

"Responsiveness" can be assessed using any endpoint indicating a benefit to the patient, including, without limitation, (1) inhibition, to some extent, of disease progression, including slowing down and complete arrest; (2) reduction in the number of disease episodes and/or symptoms; (3) reduction in lesional size; (4) inhibition (i.e., reduction, slowing down or complete stopping) of disease cell infiltration into adjacent peripheral organs and/or tissues; (5) inhibition (i.e., reduction, slowing down or complete stopping) of disease spread; (6) relief, to some extent, of one or more symptoms associated with the disorder; (7) increase in the length of disease-free presentation following treatment; (8) decreased mortality at a given point of time following treatment; and/or (9) lack of adverse effects following treatment. Responsiveness can also be assessed using any endpoint indicating side effect and/or toxicity to the patient.

"Treating" or "treatment" or "alleviation" refers to therapeutic treatment wherein the object is to slow down (lessen) if not cure the targeted pathologic condition or disorder or prevent recurrence of the condition. A subject is successfully "treated" if, after receiving a therapeutic amount of a therapeutic agent, the subject shows observable and/or measurable reduction in or absence of one or more signs and symptoms of the particular disease. For example, significant reduction in the number of cancer cells or absence of the cancer cells; reduction in the tumor size; inhibition (i.e., slow to some extent and preferably stop) of tumor metastasis; inhibition, to some extent, of tumor growth; increase in length of remission, and/or relief to some extent, one or more of the symptoms associated with the specific cancer; reduced morbidity and mortality, and improvement in quality of life issues. Reduction of the signs or symptoms of a disease may also be felt by the patient. Treatment can achieve a complete response, defined as disappearance of all signs of cancer, or a partial response, wherein the size of the tumor is decreased, preferably by more than 50 percent, more preferably by 75%. A patient is also considered treated if the patient experiences stable disease. In some embodiments, treatment with a therapeutic agent is effective to result in the patients being disease-free 3 months after treatment, preferably 6 months, more preferably one year, even more preferably 2 or more years post treatment. These parameters for assessing successful treatment and improvement in the disease are readily measurable by routine procedures familiar to a physician of appropriate skill in the art.

The term "prediction" or "prognosis" is used herein to refer to the likelihood that a patient will respond either favorably or unfavorably to a drug or set of drugs. The prediction may relate to the extent of those responses. The prediction may relate to whether and/or the probability that a patient will survive or improve following treatment, for example treatment with a particular therapeutic agent, and for a certain period of time without disease recurrence. The predictive methods of the disclosure can be used clinically to make treatment decisions by choosing the most appropriate treatment modalities for any particular patient. The predictive methods of the present disclosure are valuable tools in predicting if a patient is likely to respond favorably to a treatment regimen, such as a given therapeutic regimen, including for example, administration of a given therapeutic agent or combination, surgical intervention, steroid treatment, etc.

As used herein, the term "specifically binds" refers to the binding specificity of a specific binding pair. Recognition by an antibody of a particular target in the presence of other potential targets is one characteristic of such binding. Specific binding involves two different molecules wherein one of the molecules specifically binds with the second molecule through chemical or physical means. The two molecules are related in the sense that their binding with each other is such that they are capable of distinguishing their binding partner from other assay constituents having similar characteristics. The members of the binding component pair are referred to as ligand and receptor (anti-ligand), specific binding pair (SBP) member and SBP partner, and the like. A molecule may also be an SBP member for an aggregation of molecules; for example an antibody raised against an immune complex of a second antibody and its corresponding antigen may be considered to be an SBP member for the immune complex.

As used herein, the term "homologue" is used to refer to a nucleic acid which differs from a naturally occurring nucleic acid (i.e., the "prototype" or "wild-type" nucleic acid) by minor modifications to the naturally occurring nucleic acid, but which maintains the basic nucleotide structure of the naturally occurring form. Such changes include, but are not limited to: changes in one or a few nucleotides, including deletions (e.g., a truncated version of the nucleic acid) insertions and/or substitutions. A homologue can have enhanced, decreased, or substantially similar properties as compared to the naturally occurring nucleic acid. A homologue can be complementary or matched to the naturally occurring nucleic acid.
Homologues can be produced using techniques known in the art for the production of nucleic acids including, but not limited to, recombinant DNA techniques, chemical synthesis, etc.

As used herein, "complementary or matched" means that two nucleic acid sequences have at least 50% sequence identity. Preferably, the two nucleic acid sequences have at least 60%, 70%, 80%, 90%, 95%, 96%, 97%, 98%, 99% or 100% of sequence identity. "Complementary or matched" also means that two nucleic acid sequences can hybridize under low, middle and/or high stringency condition(s).

As used herein, "substantially complementary or substantially matched" means that two nucleic acid sequences have at least 90% sequence identity. Preferably, the two nucleic acid sequences have at least 95%, 96%, 97%, 98%, 99% or 100% of sequence identity. Alternatively, "substantially complementary or substantially matched" means that two nucleic acid sequences can hybridize under high stringency condition(s).

In general, the stability of a hybrid is a function of the ion concentration and temperature. Typically, a hybridization reaction is performed under conditions of lower stringency, followed by washes of varying, but higher, stringency. Moderately stringent hybridization refers to conditions that permit a nucleic acid molecule such as a probe to bind a complementary nucleic acid molecule. The hybridized nucleic acid molecules generally have at least 60% identity, including for example at least any of 70%, 75%, 80%, 85%, 90%, or 95% identity. Moderately stringent conditions are conditions equivalent to hybridization in 50% formamide, 5x Denhardt's solution, 5x SSPE, 0.2% SDS at 42°C, followed by washing in 0.2x SSPE, 0.2% SDS, at 42°C. High stringency conditions can be provided, for example, by hybridization in 50% formamide, 5x Denhardt's solution, 5x SSPE, 0.2% SDS at 42°C, followed by washing in 0.1x SSPE, and 0.1% SDS at 65°C.

Low stringency hybridization refers to conditions equivalent to hybridization in 10% formamide, 5x Denhardt's solution, 6x SSPE, 0.2% SDS at 22°C, followed by washing in 1x SSPE, 0.2% SDS, at 37°C. Denhardt's solution contains 1% Ficoll, 1% polyvinylpyrolidone, and 1% bovine serum albumin (BSA). 20x SSPE (sodium chloride, sodium phosphate, ethylene diamide tetraacetic acid (EDTA)) contains 3M sodium chloride, 0.2M sodium phosphate, and 0.025 M (EDTA). Other suitable moderate stringency and high stringency hybridization buffers and conditions are well known to those of skill in the art.

Other objects, advantages and features of the present invention will become apparent from the following specification taken in conjunction with the accompanying drawings.

### C. Methods for Genotyping Genomic DNA

The present invention provides a novel method to identify and verify pharmacogenomic biomarkers utilizing archived clinical samples from plasma. The present invention provides a method to identify and verify one or more pharmacogenomic biomarkers, which method comprises steps (a)-(g) as recited in claim 1.

The method may be used for retrospective study of archived clinical samples from a previously conducted clinical trial. The method may be used for *de novo* identification of a pharmacogenomic biomarker.

At least two patients exhibiting different values in a relevant phenotype are needed for the method for identifying one or more pharmacogenomic biomarkers. Typically, significantly more patients may be needed for performing the association analysis. The archived clinical samples may be from about 5, 10, 20, 50, 100, 200, 500, 1,000 or more patients. Any relevant phenotype is contemplated by the present invention, such as responsiveness to a medical treatment. The relevant phenotype may be a categorical trait, a quantitative trait or another relevant phenotype. Typically, the patients may be enrolled in a clinical trial, and their phenotype data is available. Alternatively, patients enrolled in multiple clinical trials may be pooled for the association analysis. Preferably, the multiple clinical trials relate to similar medical treatment, such as the same therapeutic agent, and data related to the relevant phenotype is available for all clinical trials.

### Sample preparation

The archived clinical samples are plasma samples.

Archived plasma samples are used to illustrate the invention. The archived plasma samples collected from patients or healthy volunteers may be used to extract DNA using any suitable method, such as the QIAGEN QIAamp MinElute Virus Spin Kit (Valencia, CA). This kit might be used with some modifications. For instance, 1 ml of plasma is vortexed briefly, and mixed thoroughly with 30 µg tRNA. The mixture is divided into 200 µl aliquots which are incubated for 1 hour before adding a lysis buffer. The lysate is then boiled for 5 minutes at 96°C and each aliquot is filtered through the same column. The DNA is eluted in 10 mM Tris-HCl (pH 8.5), vacuum-dried, and dissolved in sterile water. In most cases, the quantity of genomic DNA extracted from plasma is too low and inadequate for the subsequent genotyping. Therefore, in some embodiments, the isolated DNA may be suboptimal genomic DNA. Amplification of the isolated DNA may be needed to obtain sufficient amounts of DNA for the subsequent genotyping. In some embodiments, the amplification may be WGA, and the resulting DNA is wgaDNA. For example, DNA samples may be amplified using the Amersham Bioscience GenomiPhi DNA Amplification Kit (Piscataway, NJ) or equivalent reagents, and this process will typically yield several micrograms of DNA, which are sufficient for genotyping.

### Genotyping using SNPs

Any suitable methods may be used for obtaining genotyping data from the isolated genomic DNA. A method of genotyping may obtain information about one or more individuals at one or more polymorphic loci simultaneously. Genotyping may be defined as distinguishing alleles at a given genetic locus at single nucleotide resolution. A genetic locus is defined as a chromosomal location of a genetic or DNA marker. Thus the methods according to the present disclosure have the precision required to provide screening and diagnostic information for individuals that can be used as the basis for medical decisions.

The term "genotyped" as used herein refers to a process for determining a genotype of one or more individuals, where a "genotype" is a representation of one or more polymorphic variants in a population. Typically, genotyping involves assessing the presence or absence of polymorphic variants at one or more polymorphic loci. High-density genotyping may be used. The high-density genotyping may be whole-genome genotyping.

As used herein, the term "polymorphic locus" refers to a region in a nucleic acid at which two or more alternative nucleotide sequences are observed in a significant number of nucleic acid samples from a population of individuals. A polymorphic locus may be a nucleotide sequence of two or more nucleotides, an inserted nucleotide or nucleotide sequence, a deleted nucleotide or nucleotide sequence, or variation in the copy number of a microsatellite, for example. A polymorphic locus that is two or more nucleotides in length may be 3, 4, 5, 6, 7, 8, 9, 10, 11, 12, 13, 14, 15 or more, 20 or more, 30 or more, 50 or more, 75 or more, 100 or more, 500 or more, or about 1000 nucleotides in length, where all or some of the nucleotide sequences differ within the region. A polymorphic locus is often one nucleotide in length, which is referred to herein as a "single nucleotide polymorphism" or a "SNP." The high-density genotyping may be conducted by using SNPs. About 1,000-5,000,000 or more, preferably about 1,000,000, SNPs, may be used. The high-density genotyping may be array-based. The high-density genotyping may be conducted by sequencing, such as high-throughput sequencing.

Where there are two, three, or four alternative nucleotide sequences at a polymorphic locus, each nucleotide sequence is referred to as a "polymorphic variant" or "nucleic acid variant." Where two polymorphic variants exist, for example, the polymorphic variant represented in a minority of samples from a population is sometimes referred to as a "minor allele" and the polymorphic variant that is more prevalently represented is sometimes referred to as a "major allele." Many organisms possess a copy of each chromosome (e.g.,
humans), and those individuals who possess two major alleles or two minor alleles are often referred to as being "homozygous" with respect to the polymorphism, and those individuals who possess one major allele and one minor allele are normally referred to as being "heterozygous" with respect to the polymorphism. Individuals who are homozygous with respect to one allele are sometimes predisposed to a different phenotype as compared to individuals who are heterozygous or homozygous with respect to another allele.

In genetic analysis that identifies one or more pharmacogenomic biomarkers, samples from individuals having different values in a relevant phenotype often are allelotyped and/or genotyped. The term "allelotype" as used herein refers to a process for determining the allele frequency for a polymorphic variant in pooled DNA samples from cases and controls. By pooling DNA from each group, an allele frequency for each locus in each group is calculated. These allele frequencies are then compared to one another.

A genotype or polymorphic variant may be expressed in terms of a "haplotype," which as used herein refers to a set of DNA variations, or polymorphisms, that tend to be inherited together. A haplotype can refer to a combination of alleles or to a set of SNPs found on the same chromosome. For example, two SNPs may exist within a gene where each SNP position includes a cytosine variation and an adenine variation. Certain individuals in a population may carry one allele (heterozygous) or two alleles (homozygous) having the gene with a cytosine at each SNP position. As the two cytosines corresponding to each SNP in the gene travel together on one or both alleles in these individuals, the individuals can be characterized as having a cytosine/cytosine haplotype with respect to the two SNPs in the gene.

Researchers sometimes report a polymorphic variant in a database without determining whether the variant is represented in a significant fraction of a population. Because a subset of these reported polymorphic variants are not represented in a statistically significant portion of the population, some of them are sequencing errors and/or not biologically relevant. Thus, it is often not known whether a reported polymorphic variant is statistically significant or biologically relevant until the presence of the variant is detected in a population of individuals and the frequency of the variant is determined. A polymorphic variant is statistically significant and often biologically relevant if it is represented in 1% or more of a population, sometimes 5% or more, 10% or more, 15% or more, or 20% or more of a population, and often 25% or more, 30% or more, 35% or more, 40% or more, 45% or more, or 50% or more of a population.

A polymorphic variant may be detected on either or both strands of a double- stranded nucleic acid. Also, a polymorphic variant may be located within an intron or exon of a gene or within a portion of a regulatory region such as a promoter, a 5' untranslated region (UTR), a 3' UTR, a translated region, an intergenic region, or in a genomic region containing no known genes. Polymorphic variations may or may not result in detectable differences in gene expression, polypeptide structure, or polypeptide function.

### Parameter optimization

The genotype results derived from DNA from archived samples exhibit different characteristics, which might exhibit significantly lower call rate than that from high quality DNA used in standard practice. In contrast to applying a typical call rate cut off used in standard GWAS, which is typically 95% or even higher, the analysis used in present invention adopts an unconventional approach to adjust the cut off call rate based on the genotype results obtained in order to include more samples in the analysis. As a result, the cut off value used might be substantially lower from the standard cut off value recommended by the vendors. Another related characteristic of the results generated from this invention is that a large volume of data may be missing, and it could present significant challenge when using standard or "known" analysis algorithms. Therefore, the present invention also uses imputation algorithm that can replace some of the missing data based on linkage disequilibrium (LD) among the genotyped polymorphic loci.

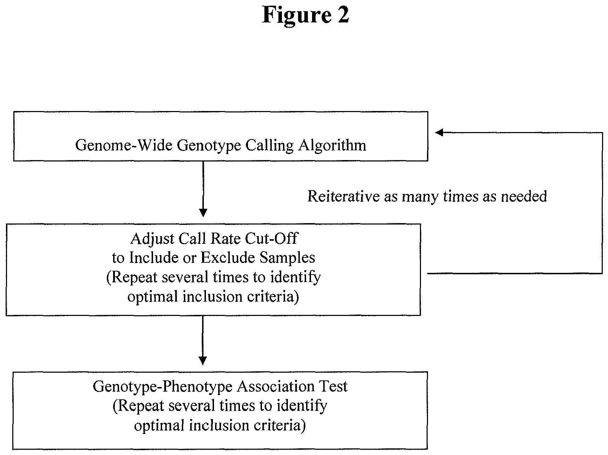

Optimization of an inclusion criterion may be performed for obtaining the genotyping data (Fig. 2). The genotyping data is obtained by using a genome-wide genotype calling algorithm. Due to the quality of the suboptimal genomic DNA generated from the plasma samples and the whole genome amplification applied prior to genotyping, the call rates from some of the samples might be substantially lower than the typical call rates (>95%) when using high quality genomic DNA. The cut-off call rate may be adjusted significantly lower than the conventional standard to include as many samples as possible. The call rate cut-off value of the genome-wide genotype calling algorithm is adjusted. The genotype calls are made by using a call rate cut-off value that is lower than a typical call rate cut-off value used for whole-genome genotyping of high quality genomic DNA, wherein the call rate cut-off value used is lower than 95%. Adjustment of the call rate cut-off value of the genotype calling algorithm and making the genotype calls based on the adjusted call rate cut-of value may be repeated multiple times to identify a criterion to include and/or exclude samples. An optimal inclusion criterion, such as call rate cut-off value, may be identified after iterations of the adjustment cycle. In some embodiments, the genotype calls are made using an imputation algorithm, wherein the HapMap is used for the imputation algorithm.

In some embodiments, the DNA samples may be genotyped using whole genome SNP arrays manufactured by Affymetrix (Santa Clara, CA) and/or Illumina (San Diego, CA). Affymetrix 500K array is used as an example to illustrate the present invention. In addition to Affymetrix arrays, Illumina chips and Sequenom MassArray are also used to confirm the results generated by one platform.

The genotype calls may be generated with the Affymetrix Genotyping Console™ software. The genotype calls may be generated using the Robust Linear Model with the Mahalanobis Distance Classifier (RLMM) algorithm, the RLMM with a Bayesian step (BRLMM) algorithm, the Axiom™ GT1 algorithm, the BRLMM using perfect-match probes (BRLMM-P) algorithm, or the Birdseed algorithm (Rabbee et al., Bioinformatics (2006) 22:7-12; Korn et al, Nat Genet (2008) 40:1253-60).

### D. Identifying Pharmacogenomic Biomarkers by Association Analysis

The genotyping data obtained are used for performing association analysis based on the relevant phenotype to identify pharmacogenomic biomarkers (Fig. 1). Classification algorithms, which include but are not limited to support vector machine (SVM) and logistic regression, may be applied to the dataset and identify the optimal biomarkers and scoring algorithm. The association analysis may be a GWAS. The association analysis may be performed by calculating an associated p- value of each polymorphic locus, such as SNP, with the relevant phenotype. The calculation may be based on an allele frequency and/or genotype-based test.

The relevant phenotypes for identifying the pharmacogenomic biomarkers are generally related to responsiveness of individuals to a treatment regimen. A relevant phenotype may be qualitative or quantitative. Responsiveness may be primary, such as reduce in cancer mass in response to an anti-cancer drug, or secondary, such as hypertriglyceridemia (HTC) in response to bexarotene. The primary and secondary responses may or may not be correlated with each other. A response may be positive or negative. A negative response may be defined as either the absence of an efficacious response or the presence of toxic side effects. One or more relevant phenotypes may be used for the association analysis to identify the pharmacogenomic biomarkers.

The association analysis may be repeated multiple times using different call rate cut-off values to optimize criteria used (Fig. 2). The genotype results may be analyzed using genetic data analysis software such as PLINK (Purcell et al. Am J Hum Genet (2007) 81:559-5752) to calculate associated p-value of each polymorphic locus with the relevant phenotype. Polymorphic loci may be ordered by their calculated association with the relevant phenotype. The most significantly associated polymorphic loci may be identified as pharmacogenomic biomarkers for the relevant phenotype.

### Verification and/or replication

The pharmacogenomic biomarkers identified by high-density genotyping are subject to further association analyses, or the "Stage II" analyses (Fig. 1). The further association analyses are used for verification and /or replication of the identified pharmacogenomic biomarkers through high-density genotyping and association analysis, or the Stage I analysis. Some or all of the archived clinical samples from Stage I and/or additional clinical samples may be used for the additional genotyping.

The further association analysis may be based on additional genotyping data using the identified pharmacogenomic biomarkers. About 1, 10, 20, 50, 100, 200, 500 or more of the identified pharmacogenomic biomarkers may be used for the additional genotyping.

The highly associated SNPs identified from the Stage I analysis may be replicated with low density genotyping platforms which could be distinct from those used in Stage I, such as Sequenom iPLEX MassArray technology.

In Stage II, new DNA samples, which have not been used in Stage I, may be genotyped arming to replicate the pharmacogenomic biomarker identified in Stage I.

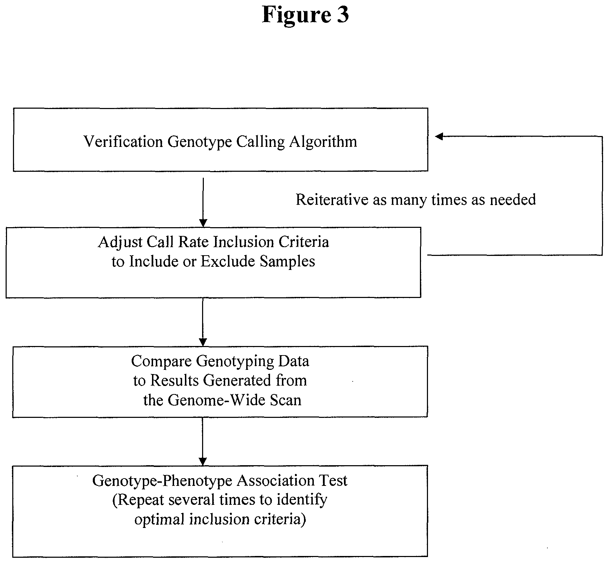

Optimization of an inclusion criterion is performed for obtaining the additional genotyping data (Fig. 3). The additional genotyping data are obtained by using a verification genotype calling algorithm. The further association analysis comprises adjusting the call rate cut-off value of the verification genotype calling algorithm. Adjusting the call rate cut-off is repeated multiple times to include and/or exclude samples. The method further comprises comparing the additional genotyping data obtained by using the verification genotype calling algorithm to the genotyping data obtained by using the genome- wide genotype calling algorithm (Fig. 3).

After the two-stage studies, the most significantly associated pharmacogenomic biomarkers can be identified. The association analysis may be repeated multiple times using different call rate cut-off values to optimize criteria used (Fig. 3). The pharmacogenomic biomarkers may be a subgroup of the pharmacogenomic biomarkers identified in Stage I. The pharmacogenomic biomarkers may comprise one or more SNPs. Multiple phannacogenomic biomarkers identified may be located close to each other on the genome, and may locate in an intron/exon of a gene, an intergenic region, or a region containing no known gene on the genome.

### E. Genome-Wide Association Study Using Archived Samples

Provided herein is a method to conduct GWAS using suboptimal genomic DNA. The suboptimal genomic DNA may be from archived samples. The suboptimal genomic DNA may be from plasma samples. The suboptimal genomic DNA may be amplified. Further provided herein is a method for identifying a phannacogenomic biomarker using the method to conduct GWAS using suboptimal genomic DNA, wherein the method may be a retrospective method.

The two-stage data analysis described above may be used for the GWAS using suboptimal genomic DNA. Multiple genotvping platforms may be used. The same or different samples may be used for the multiple genotyping platforms. The method may further use a sample providing high-quality genomic DNA, which may be used for replication of the data obtained from suboptimal genomic DNA. The samples providing high-quality genomic DNA may be whole blood samples.

### F. Applications of the Pharmacogenomic Biomarkers

Pharmacogenomics involves tailoring a treatment for a subject according to the subject's genotype as a particular treatment regimen may exert a differential effect depending upon the subject's genotype. For example, based upon the outcome of a prognostic test, a clinician or physician may target pertinent information and preventative or therapeutic treatments to a subject who would be benefited by the information or treatment and avoid directing such information and treatments to a subject who would not be benefited (e.g., the treatment has no therapeutic effect and/or the subject experiences adverse side effects). Information generated from pharmacogenomic biomarkers using a method described herein can be used to determine appropriate dosage and treatment regimens for an individual. This knowledge, when applied to dosing or drag selection, can avoid adverse reactions or therapeutic failure and thus enhance therapeutic efficiency when administering a therapeutic composition. In some embodiments, the pharmacogenomic biomarker may be used to develop a companion diagnostic test.

Therefore, provided herein is a companion diagnostic test using the pharmacogenomic biomarkers identified by the method disclosed herein. For example, a physician or clinician may consider applying knowledge obtained in pharmacogenomic biomarkers using a method described herein, when determining whether to administer a pharmaceutical composition to a subject. A physician or clinician may consider applying such knowledge when determining the dosage, e.g., amount per treatment or frequency of treatments, of a treatment, administered to a patient.

The disclosure provides methods for assessing or aiding assessment of responsiveness of a subject to treatment. The disclosure also provides methods for predicting responsiveness or monitoring treatment/responsiveness to a treatment in a subject. The disclosure provides methods for selecting a subject for treatment and treating the subject. The methods may comprise assessing one or more pharmacogenomic biomarkers in a sample obtained from the subject; and predicting, assessing, or aiding assessment of responsiveness of the subject to a treatment based on the genotype of said one or more pharmacogenomic biomarkers. The responsiveness may be predicted or assessed by classifying the subject using an algorithm such as SVM, logistic regression, or K-nearest neighbors analysis.

The following is an example of a pharmacogenomic variant. A particular treatment regimen can exert a differential effect depending upon the subject's genotype. Where a candidate therapeutic exhibits a significant interaction with a major allele and a comparatively weak interaction with a minor allele (e.g., an order of magnitude or greater difference in the interaction), such a therapeutic typically would not be administered to a subject genotyped as being homozygous for the minor allele, and sometimes not administered to a subject genotyped as being heterozygous for the minor allele. In another example, where a candidate therapeutic is not significantly toxic when administered to subjects who are homozygous for a major allele but is comparatively toxic when administered to subjects heterozygous or homozygous for a minor allele, the candidate therapeutic is not typically administered to subjects who are genotyped as being heterozygous or homozygous with respect to the minor allele.

The methods described herein are applicable to pharmacogenomic methods for preventing, alleviating or treating conditions such as metabolic disorders, cardiovascular diseases, cancers, etc. For example, a nucleic acid sample from an individual may be subjected to a prognostic test described herein. Where one or more polymorphic variations associated with increased risk of type II diabetes are identified in a subject, information for preventing or treating type II diabetes and/or one or more type II diabetes treatment regimens then may be prescribed to that subject.

A treatment regimen may be specifically prescribed and/or administered to individuals who will most benefit from it based upon their likelihood of responding to a treatment regimen assessed by the methods described herein. Thus, provided are methods for identifying a subject with a high likelihood of responding to a treatment regimen and then prescribing such treatment regimen to individuals identified as having a high likelihood of responding. Thus, certain variants are directed to a method for treating a subject, which comprises: detecting the presence or absence of a pharmacogenomic biomarker associated with responsiveness to a treatment regimen in a nucleotide sequence set forth herein in a nucleic acid sample from a subject, and prescribing or administering the treatment regimen to a subject from whom the sample originated where the presence of a pharmacogenomic biomarker associated with responsiveness to the treatment regimen is detected in the nucleotide sequence.

The treatment sometimes is preventative (e.g., is prescribed or administered to reduce the probability that a disease condition arises or progresses), sometimes is therapeutic, and sometimes delays, alleviates or halts the progression of a disease condition. Any known preventative or therapeutic treatment for alleviating or preventing the occurrence of a disorder may be prescribed and/or administered.

Pharmacogenomics methods also may be used to analyze and predict a response to a drug. For example, if pharmacogenomics analysis indicates a likelihood that an individual will respond positively to a treatment with a particular drug, the drug may be administered to the individual. Conversely, if the analysis indicates that an individual is likely to respond negatively to treatment with a particular drug, an alternative course of treatment may be prescribed. The response to a therapeutic treatment can be predicted in a background study in which subjects in any of the following populations are genotyped: a population that responds favorably to a treatment regimen, a population that does not respond significantly to a treatment regimen, and a population that responds adversely to a treatment regiment (e.g., exhibits one or more side effects). These populations are provided as examples and other populations and subpopulations may be analyzed. Based upon the results of these analyses, a subject is genotyped to predict whether he or she will respond favorably to a treatment regimen, not respond significantly to a treatment regimen, or respond adversely to a treatment regimen.

A classification/prediction algorithm may be developed using the verification and/or replication dataset. An imputation algorithm that can replace some of the missing data based on LD among the genotyped polymorphic loci may be used. In variants where SNPs are used for genotyping, SNP databases such as Hapmap may be used for the imputation algorithm. For development of the classification/prediction algorithm, the verification dataset may be used as a training dataset. Once a classification/prediction algorithm has been developed, the replication dataset may be used for testing the algorithm.

The methods of the disclosure may comprise classifying the subject as a responsive or non-responsive subject using a K-nearest neighbors analysis based on the genotype of the pharmacogenomic biomarkers in the sample from the subject and reference samples with known classes. Classifying the subject using a K-nearest neighbors analysis may be carried out by (1) determining parameter K (i.e., number of nearest neighbors); (2) calculating the difference between the measured expression level of the marker genes in the new sample to be classified and the expression level of the respective marker genes in each reference sample; (3) determining the nearest reference samples by selecting those samples with the smallest weighted average of the absolute differences (WAAD) between the new sample and the reference sample; and (4) determining class of the new sample based on the known classes of the K nearest reference samples. The weights and/or parameter K are determined using cross-validation with clinical trial samples with known classes. For example, 5-fold (such as 5-fold, 6-fold, 7-fold, 8-fold, 9-fold, or 10-fold) to N-fold cross-validation may be used to minimize the weighted K-nearest neighbors classification error, wherein N is the size of the samples. K may be an integer between 4 and 13 (e.g., 4, 5, 6, 7, 8, 9, 10, 11, 12, and 13). The nearest reference samples (nearest neighbors) may be those with the smallest weighted average of the absolute differences between the expression level of the new sample to be classified and the expression level of each reference sample for each of the pharmacogenomic biomarkers.

The comparisons and/or calculations for predicting, assessing or aiding assessment can be carried out in any convenient manner appropriate to the type of measured value and/or reference value for the pharmacogenomic biomarkers at issue. The process of comparing or calculating may be manual or it may be automatic (such as by a machine including computer-based machine). As will be apparent to those of skill in the art, replicate genotyping may be taken for the pharmacogenomic biomarkers.

Also provided herein is a method of prognosticating responsiveness of a subject to a treatment using the companion diagnostic test disclosed herein. The tests described herein also are applicable to clinical drug trials. The pharmacogenomic biomarkers can be used to stratify or select a subject population for a clinical trial. The pharmacogenomic biomarkers can be used to stratify individuals that may exhibit a toxic response to a treatment from those that will not. The pharmacogenomic biomarkers can be used to separate those that will be non-responders from those who will be responders. The pharmacogenomic biomarkers described herein can be used in pharmacogenomic-based design and in managing the conduct of a clinical trial.

One or more pharmacogenomic biomarkers indicative of response to a therapeutic agent or side effects to a therapeutic agent may be identified using the methods described herein. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

Thus, also disclosed is a method of selecting an individual for inclusion in a clinical trial of a treatment or drug comprising the steps of: (a) obtaining a nucleic acid sample from an individual; (b) determining the identity of a polymorphic variation which is associated with a positive response to the treatment or the drug, or at least one polymorphic variation which is associated with a negative response to the treatment or the drug in the nucleic acid sample, and (c) including the individual in the clinical trial if the nucleic acid sample contains said polymorphic variation associated with a positive response to the treatment or the drug or if the nucleic acid sample lacks said polymorphic variation associated with a negative response to the treatment or the drug. In addition, the methods described herein for selecting an individual for inclusion in a clinical trial of a treatment or drug encompass methods with any further limitation described in this disclosure, or those following, specified alone or in any combination. The including step (c) optionally comprises administering the drug or the treatment to the individual if the nucleic acid sample contains the polymorphic variation associated with a positive response to the treatment or the drug and the nucleic acid sample lacks said biallelic marker associated with a negative response to the treatment or the drug.

### G. Additional Pharmacogenomic Biomarkers or Drug Targets

Also provided is a method for identifying polymorphic variants proximal to an identified pharmacogenomic biomarker. Thus, featured herein are methods for identifying a polymorphic variation that is proximal to an identified pharmacogenomic biomarker. The proximal polymorphic variant identified sometimes may be a publicly disclosed polymorphic variant, which for example, sometimes is published in a publicly available database. The polymorphic variant identified may be not publicly disclosed and is discovered using a known method, including, but not limited to, sequencing a region surrounding the identified pharmacogenomic biomarker in a group of nucleic samples. Thus, multiple polymorphic variants proximal to an identified pharmacogenomic biomarker are identified using this method.

The proximal polymorphic variant often is identified in a region surrounding the identified pharmacogenomic biomarker. This surrounding region may be about 50 kb flanking the identified pharmacogenomic biomarker (e.g., about 50 kb 5' of the first polymorphic variant and about 50 kb 3' of the first polymorphic variant), and the region sometimes is composed of shorter flanking sequences, such as flanking sequences of about 40 kb, about 30 kb, about 25 kb, about 20 kb, about 15 kb, about 10 kb, about 7 kb, about 5 kb, or about 2 kb 5' and 3' of the identified pharmacogenomic biomarker. The region may be composed of longer flanking sequences, such as flanking sequences of about 55 kb, about 60 kb, about 65 kb, about 70 kb, about 75 kb, about 80 kb, about 85 kb, about 90 kb, about 95 kb, or about 100 kb 5' and 3' of the identified pharmacogenomic biomarker.

The pharmacogenomic biomarkers may be used to identify one or more additional pharmacogenomic biomarkers. For example, other polymorphic loci located in proximity to the pharmacogenomic biomarkers may be analyzed for association with the relevant phenotype. Additionally, genes may be identified that are in proximity to the pharmacogenomic biomarkers, and their functions analyzed. Genes with functions that are directly or indirectly related to the relevant phenotype, or other genes in the same cellular pathway, may be targets for further analysis with the relevant phenotype, and new pharmacogenomic biomarkers may be identified.

Polymorphic variants may be identified iteratively. For example, a first proximal polymorphic variant is identified using the methods described above and then another polymorphic variant proximal to the first proximal polymorphic variant is identified (e.g., publicly disclosed or discovered) and the presence or absence of an association of one or more other polymorphic variants proximal to the first proximal polymorphic variant is determined.

The methods described herein are useful for identifying or discovering additional polymorphic variants that may be used to further characterize a gene, region or loci associated with a condition, a disease, or a disorder. For example, allelotyping or genotyping data from the additional polymorphic variants may be used to identify a functional mutation or a region of linkage disequilibrium. Polymorphic variants identified or discovered within a region comprising the identified pharmacogenomic biomarker may be genotyped using the genetic methods and sample selection techniques described herein, and it can be determined whether those polymorphic variants are in linkage disequilibrium with the identified pharmacogenomic biomarker. The size of the region in linkage disequilibrium with the identified pharmacogenomic biomarker also can be assessed using these genotyping methods. Thus, provided herein are methods for determining whether a polymorphic variant is in linkage disequilibrium with an identified pharmacogenomic biomarker, and such information can be used in prognosis/diagnosis methods described herein.

Further provided herein is a method of identifying a novel drug target using the pharmacogenomic biomarkers identified by the method disclosed herein. Said biomarkers and their associated SNPs or genes could gain insight of the underlying biological pathways or mechanisms underlying the relevant phenotypes, such as efficacy, adverse effect, or other endpoints. These discoveries could be instrumental for developing better diagnosis or therapeutic agents.

### H. Kits

Diagnostic kits based on the pharmacogenomic biomarker described above might be developed, and they can be used to predict individual's response to the corresponding drug. Such test kits can include devices and instructions that a subject can use to obtain a sample, e.g., of buccal cells or blood, without the aid of a health care provider.

For use in the applications described or suggested above, kits or articles of manufacture are also provided herein. Such kits may comprise at least one reagent specific for genotyping a pharmacogenomic biomarker described herein, and may further include instructions for carrying out a method described herein.

The disclosure provides compositions and kits comprising primers and primer pairs, which allow the specific amplification of the polynucleotides or of any specific parts thereof, and probes that selectively or specifically hybridize to nucleic acid molecules or to any part thereof. Probes may be labeled with a detectable marker, such as, for example, a radioisotope, fluorescent compound, bioluminescent compound, a chemiluminescent compound, metal chelator or enzyme. Such probes and primers can be used to detect the presence of polynucleotides in a sample and as a means for detecting cell expressing proteins encoded by the polynucleotides. As will be understood by the skilled artisan, a great many different primers and probes may be prepared based on the sequences provided herein and used effectively to amplify, clone and/or determine the presence and/or levels of genomic DNAs.

The kit may comprise reagents for detecting presence of polypeptides. Such reagents may be antibodies or other binding molecules that specifically bind to a polypeptide. Such antibodies or binding molecules may be capable of distinguishing a structural variation to the polypeptide as a result of polymorphism, and thus may be used for genotyping. The antibodies or binding molecules may be labeled with a detectable marker, such as, for example, a radioisotope, a fluorescent compound, a bioluminescent compound, a chemiluminescent compound, a metal chelator, an enzyme, or a particle. Other reagents for performing binding assays, such as ELISA, may be included in the kit.

The kits comprise reagents for genotyping at least two, at least three, at least five, at least ten, or fifteen pharmacogenomic biomarkers. The kits may further comprise a surface or substrate (such as a microarray) for capture probes for detecting of amplified nucleic acids.

The kits may further comprise a carrier means being compartmentalized to receive in close confinement one or more container means such as vials, tubes, and the like, each of the container means comprising one of the separate elements to be used in the method. For example, one of the container means may comprise a probe that is or can be detectably labeled. Such probe may be a polynucleotide specific for a pharmacogenomic biomarker. Where the kit utilizes nucleic acid hybridization to detect the target nucleic acid, the kit may also have containers containing nucleotide(s) for amplification of the target nucleic acid sequence and/or a container comprising a reporter-means, such as a biotin-binding protein, such as avidin or streptavidin, bound to a reporter molecule, such as an enzymatic, florescent, or radioisotope label.

The kit will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific therapy or non-therapeutic application, and may also indicate directions for either in vivo or in vitro use, such as those described above.

The kit can further comprise a set of instructions and materials for preparing a tissue or cell sample and preparing nucleic acids (such as genomic DNA) from the sample.

The disclosure provides a variety of compositions suitable for use in performing methods of the disclosure, which may be used in kits. For example, the disclosure provides surfaces, such as arrays that can be used in such methods. An array comprises individual or collections of nucleic acid molecules useful for detecting pharmacogenomic biomarkers. For instance, an array may comprises a series of discretely placed individual nucleic acid oligonucleotides or sets of nucleic acid oligonucleotide combinations that are hybridizable to a sample comprising target nucleic acids, whereby such hybridization is indicative of genotypes of the pharmacogenomic biomarkers.

Several techniques are w ell-known in the art for attaching nucleic acids to a solid substrate such as a glass slide. One method is to incorporate modified bases or analogs that contain a moiety that is capable of attachment to a solid substrate, such as an amine group, a derivative of an amine group or another group with a positive charge, into nucleic acid molecules that are synthesized. The synthesized product is then contacted with a solid substrate, such as a glass slide, which is coated with an aldehyde or another reactive group which will form a covalent link with the reactive group that is on the amplified product and become covalently attached to the glass slide. Other methods, such as those using amino propryl silica surface chemistry are also known in the art, as disclosed at world wide web at cmt.corning.com and cmgm.stanford.edu/pbrownl.

Attachment of groups to oligonucleotides which could be later converted to reactive groups is also possible using methods known in the art. Any attachment to nucleotides of oligonucleotides will become part of oligonucleotide, which could then be attached to the solid surface of the microarray. Amplified nucleic acids can be further modified, such as through cleavage into fragments or by attachment of detectable labels, prior to or following attachment to the solid substrate, as required and/or permitted by the techniques used.

The present disclosure can be applied broadly in the biomedical fields and offers a number of major advantages to modem drug development and the emerging field of personalized medicine. These benefits include, but are not limited to, shortening the time and cutting the cost required to identify biomarkers for drugs in clinical development, drastically enhancing the chance of success for investigated drugs, rescuing drags which would have been abandoned without patient stratification in clinical trials.

### I. Computer Readable Medium

In yet another aspect, provided herein is a computer readable medium comprising a plurality of instructions for a genotyping method using suboptimal genomic DNA samples, which comprises the steps of: a) receiving sequence information of said suboptimal genomic DNA samples; b) optimizing an inclusion criterion based on said sequence information; and c) calculating genotypes based on said sequence information and said optimized inclusion criterion.

Also provided herein is a genotyping method using suboptimal genomic DNA samples, which method comprises optimizing an inclusion criterion. The optimization may be repeated multiple times to include and/or exclude samples. An optimal inclusion criterion may be identified. The genotyping data may be obtained by using a genome-wide genotype calling algorithm and/or a verification genotype calling algorithm. The inclusion criterion may be the call rate cut-off value of the genotype calling algorithm. In some embodiments, the genotype calls may be made by using a call rate cut-off that is lower than a typical call rate cut-off used for whole-genome genotyping of high quality genomic DNA, wherein the call rate cut-off value used may be about 50%, 60%, 70%, 80%, 90% or 95%. The genotyping data may be obtained by using multiple genotyping platforms. The genotyping data from multiple genotyping platforms may be compared for the optimization.

Further provided is a method to perform association analysis using the genotyping method using suboptimal genomic DNA samples, which method comprises optimizing an inclusion criterion. The association analysis may be repeated multiple times for the optimization.

### J. Examples

The following examples are offered to illustrate but not to limit the invention.

### Example 1

### Retrospective De Novo Identification of Pharmacogenomic Biomarkers Using Archived Plasma Samples from Clinical Trials

*Patients.* Among patients enrolled in the clinical trials and treated with the drug, plasma samples from 400 individuals are available. The cases are defined as those who responded positively from the drug treatment, and the controls are those who had no response or responded negatively from the drug treatment. Prior to the study, patient identification and individually identifiable information were removed, and all samples were relabeled by a third party to protect patient identity.

*DNA preparation.* DNA is extracted from plasma samples with the QIAGEN QIAamp MinElute Virus Spin Kit (Valencia, CA, USA) with some modifications. Briefly, 1 ml of plasma is vortexed briefly, and mixed thoroughly with 30 µg tRNA. The mixture is divided into 200 µl aliquots which are incubated for 1 hour before adding a lysis buffer. The lysate is then boiled for 5 minutes at 96°C and each aliquot is filtered through the same column. The DNA is eluted in 10 mM Tris-HCi (pH 8.5), vacuum-dried, and dissolved in sterile water. In most cases, the quantity of genomic DNA extracted from plasma is too low and inadequate for the subsequent genotyping, and DNA samples are then amplified using the Amersham Bioscience GenomiPhi DNA Amplification Kit (Piscataway, NJ, USA).

*SNP genotyping and data analysis,* In Stage I (Fig. 1), 150 samples (75 cases and 75 controls) are genotyped using the Affymetrix GeneChip 500K Mapping Array Set containing 500,000 SNPs following Affymetrix standard protocol (Santa Clara, CA, USA). Due to the quality of the suboptimal genomic DNA generated from the plasma samples and
the whole genome amplification applied prior to genotyping, the call rates from some of the samples might be substantially lower than the typical call rates (>95%) when using high quality genomic DNA. Therefore, the cut-off call rate is adjusted significantly lower than the conventional standard to include as many samples as possible. The adjustment of the call rate cut-off value of the genotype calling algorithm and making the genotype calls based on the adjusted call rate cut-of value are repeated multiple times to identify an optimal criterion to include and/or exclude samples (Fig. 2). After removing the samples having call rates lower than the optimal cut-off criteria, the genotype results are analyzed using genetic data analysis software PLINK (Purcell et al., Am J Hum Genet (2007) 81:559-5752) to calculate associated p-value of each polymorphic locus with the relevant phenotype. Polymorphic loci are ordered by their calculated association with the relevant phenotype. The 200 most significantly associated SNPs are selected for Stage II study using Sequenom iPLEX assays (Sequenom, San Diego, CA, USA). These assays are used to genotype all 400 DNA samples from the clinical trials. Among them, 150 samples used in Stage I are selected as the verification group, and the other 250 samples are used as the replication group (Fig. 1). Final genotype calls are generated by Sequenom Typer Analyzer from the MassARRAY Typer suite (Sequenom, San Diego, CA, USA). The genotyped results from the verification group are compared to the results generated in Stage I. The call rates from some of the samples might be substantially lower than the typical call rates (>95%) when using high quality genomic DNA, and the cut-off call rate is adjusted multiple times to include and/or exclude samples. After removing the samples having call rates lower than the optimal cut-off criteria and samples with too many discrepancies between the two stages (Fig. 3), association analysis is performed by calculating an associated p-value of each SNP with the relevant phenotype using PLINK program. The calculation may be based on an allele frequency and/or genotype-based test, and the relevant phenotype may be a categorical trait, a quantitative trait or another relevant phenotype. Pharmacogenomic biomarkers showing significant associations with the drug response are identified, and these pharmacogenomic biomarkers may comprise one or more SNPs. Multiple pharmacogenomic biomarkers identified may be located close to each other on the genome, and may locate in an intron/exon of a gene, an intergenic region, or a region containing no known gene on the genome. Classification algorithms, such as support vector machine (SVM) and logistic regression, may be applied to the dataset to identify the optimal biomarkers and scoring algorithm, so the patient's response to drug treatment can be properly predicted.

The above examples are included for illustrative purposes only and are not intended to limit the scope of the invention.

## Claims

1. A method to identify and verify one or more pharmacogenomic biomarkers, which method comprises:
a) isolating DNA from archived clinical samples, from a previously conducted clinical trial, of five or more patients exhibiting different values in a relevant phenotype;
b) amplifying said isolated DNA, wherein the amplification is whole-genome amplification (WGA), and the resulting DNA is whole-genome amplified DNA (wgaDNA);
c) obtaining high-density genotyping data of said amplified DNA by using a genome-wide genotype calling algorithm, wherein the call rate cut-off value of the genome-wide genotype calling algorithm is adjusted multiple times to include or exclude samples, whereby the call rate cut-off value is optimized based on sequence information from said amplified DNA, the optimized call rate cut-off value is less than 95%, and genotypes are calculated based on the sequence information and the optimized call rate cut-off value;
d) performing association analysis based on said genotyping data and said different values in said relevant phenotype to identify one or more pharmacogenomic biomarkers of said relevant phenotype,
e) obtaining additional genotyping data from archived clinical samples of additional patients from the previously conducted clinical trial by using a verification genotype calling algorithm, wherein the call rate cut-off value of the verification genotype calling algorithm is adjusted multiple times to include or exclude samples,
f) comparing the additional genotyping data obtained by using the verification genotype calling algorithm to the genotyping data obtained by using the genome-wide genotype calling algorithm, and
g) performing association analysis based on said additional genotyping data to verify association between the one or more pharmacological biomarkers identified in step d) and said relevant phenotype,
wherein a subset of the pharmacogenomic biomarkers identified in step d) is verified in step g), for retrospective study of archived clinical samples from the previously conducted clinical trial,
wherein the archived clinical samples are plasma samples, and
wherein the isolated DNA is suboptimal genomic DNA which is inferior in quality and/or in quantity to genomic DNA isolated from whole blood.

2. The method of claim 1, wherein the call rate cut-off value of the genome-wide genotype calling algorithm is adjusted to include more of the archived clinical samples.

3. The method according to claim 1, wherein the genome-wide genotype calling algorithm is an imputation algorithm.

## Patentansprüche

1. Verfahren zur Identifizierung und Verifizierung von einem oder mehreren pharmakogenomischen Biomarkern, wobei das Verfahren folgendes aufweist:
a) Isolieren von DNA aus archivierten klinischen Proben von einer zuvor durchgeführten klinischen Studie von fünf oder mehr Patienten, die unterschiedliche Werte in einem relevanten Phänotyp aufweisen;
b) Amplifizieren der isolierten DNA, wobei die Amplifikation eine vollständige Genomamplifikation (WGA) ist und die resultierende DNA eine vollständig amplifizierte genomische DNA (wgaDNA) ist;
c) Erhalten von hochdichten Genotypisierungsdaten von der amplifizierten DNA durch Verwendung eines Algorithmus zum genomweiten Genotypabruf, wobei der Cut-Off-Wert der Abrufrate des Algorithmus zum genomweiten Genotypabruf mehrere Male angepasst wird, um Proben einzuschließen oder auszuschließen, wobei der Cut-Off-Wert der Abrufrate auf der Basis von Sequenzinformation von der amplifizierten DNA optimiert wird, wobei der optimierte Cut-Off-Wert der Abrufrate kleiner als 95% ist, und die Genotypen auf der Basis der Sequenzinformation und des optimierten Cut-Off-Wertes der Abrufrate berechnet werden;
d) Durchführen einer Assoziationsanalyse basierend auf den Genotypisierungsdaten und den unterschiedlichen Werten in dem relevanten Phänotyp, um einen oder mehrere pharmakogenomische Biomarker des relevanten Phänotyps zu identifizieren,
e) Erhalten von zusätzlichen Genotypisierungsdaten aus archivierten klinischen Proben von zusätzlichen Patienten aus der zuvor durchgeführten klinischen Studie durch Verwendung eines verifizierenden Algorithmus zum Genotypabruf, wobei der Cut-Off-Wert der Abrufrate des verifizierenden Algorithmus zum Genotypabruf mehrere Male angepasst wird, um Proben einzuschließen oder auszuschließen,
f) Vergleichen der zusätzlichen Genotypisierungsdaten, die durch die Verwendung des verifizierenden Algorithmus zum Genotypabruf erhalten wurden, mit den Genotypisierungsdaten, die durch die Verwendung des Algorithmus zum genomweiten Genotypabruf erhalten wurden, und
g) Durchführen einer Assoziationsanalyse basierend auf den zusätzlichen Genotypisierungsdaten, um die Assoziation zwischen dem einen oder mehreren Biomarkern zu verifizieren, die in Schritt d) erhalten wurden, und des relevanten Phänotyps,
wobei eine Untermenge der in Schritt d) identifizierten pharmakogenomischen Biomarker in Schritt g) verifiziert wird, und zwar für eine retrospektive Untersuchung der archivierten klinischen Proben aus der zuvor durchgeführten klinischen Studie,
wobei die archivierten klinischen Proben Plasmaproben sind, und
wobei die isolierte DNA suboptimale genomische DNA ist, die hinsichtlich der Qualität und/oder Quantität gegenüber genomischer DNA, die aus Gesamtblut isoliert wurde, minderwertig ist.

2. Verfahren nach Anspruch 1, wobei der Cut-Off-Wert der Abrufrate des Algorithmus zum genomweiten Genotypabruf angepasst wird, um mehr der archivierten klinischen Proben einzuschließen.

3. Verfahren gemäß Anspruch 1, wobei der Algorithmus zum genomweiten Genotypabruf ein Anrechnungsalgorithmus ist.

## Revendications

1. Procédé pour identifier et vérifier un ou plusieurs biomarqueur(s) pharmacogénomique(s), lequel procédé comprend le fait :
a) d'isoler l'ADN à partir d'échantillons cliniques archivés, provenant d'un essai clinique précédemment mené, de cinq patients ou plus présentant des valeurs différentes dans un phénotype pertinent ;
b) d'amplifier ledit ADN isolé, où l'amplification est une amplification du génome entier (WGA), et l'ADN résultant est un ADN obtenu par amplification du génome entier (ADNwga) ;
c) d'obtenir des données de génotypage à haute densité dudit ADN amplifié en utilisant un algorithme d'appel de génotype à l'échelle du génome, où la valeur seuil de taux d'appel de l'algorithme d'appel de génotype à l'échelle du génome est ajustée plusieurs fois pour inclure ou exclure des échantillons, moyennant quoi la valeur seuil de taux d'appel est optimisée sur la base d'informations de séquence provenant dudit ADN amplifié, la valeur seuil de taux d'appel optimisée est inférieure à 95%, et les génotypes sont calculés sur la base des informations de séquence et de la valeur seuil de taux d'appel optimisée ;
d) de réaliser une analyse d'association sur la base desdites données de génotypage et desdites valeurs différentes dans ledit phénotype pertinent pour identifier un ou plusieurs biomarqueur(s) pharmacogénomique(s) dudit phénotype pertinent,
e) d'obtenir des données de génotypage supplémentaires à partir d'échantillons cliniques archivés de patients supplémentaires provenant de l'essai clinique précédemment mené en utilisant un algorithme d'appel de génotype de vérification, où la valeur seuil de taux d'appel de l'algorithme d'appel de génotype de vérification est ajustée plusieurs fois pour inclure ou exclure des échantillons,
f) de comparer les données de génotypage supplémentaires obtenues en utilisant l'algorithme d'appel de génotype de vérification aux données de génotypage obtenues en utilisant l'algorithme d'appel de génotype à l'échelle du génome, et
g) de réaliser une analyse d'association sur la base desdites données de génotypage supplémentaires pour vérifier l'association entre le ou les plusieurs biomarqueur(s) pharmacologique(s) identifié(s) à l'étape d) et ledit phénotype pertinent,
dans lequel un sous-ensemble des biomarqueurs pharmacogénomiques identifiés à l'étape d) est vérifié à l'étape g), pour l'étude rétrospective d'échantillons cliniques archivés provenant de l'essai clinique précédemment mené,
dans lequel les échantillons cliniques archivés sont des échantillons de plasma, et
dans lequel l'ADN isolé est de l'ADN génomique sous-optimal qui est de qualité et/ou de quantité inférieure(s) à l'ADN génomique isolé à partir du sang entier.

2. Procédé de la revendication 1, dans lequel la valeur seuil de taux d'appel de l'algorithme d'appel de génotype à l'échelle du génome est ajustée pour inclure plus d'échantillons cliniques archivés.

3. Procédé selon la revendication 1, dans lequel l'algorithme d'appel de génotype à l'échelle du génome est un algorithme d'imputation.
